# EUROPEAN PATENT SPECIFICATION

(11) **EP 3 040 103 B1**
(45) Date of publication and mention of the grant of the patent: **10.07.2019**
(21) Application number: 14841160.6
(22) Date of filing: 30.06.2014
(51) Int. Cl.: A61N 1/36, A61N 1/05, A61N 1/06

(54) **HIGH FREQUENCY ELECTROMAGNETIC FIELD STIMULATOR FOR CHANGING NERVE THRESHOLD**
HOCHFREQUENTER ELEKTROMAGNETFELD-STIMULATOR ZUR ÄNDERUNG EINES NERVENSCHWELLENWERTES
STIMULATEUR DE CHAMP ÉLECTROMAGNÉTIQUE À HAUTE FRÉQUENCE POUR MODIFIER LE SEUIL NERVEUX

(30) Priority: 26.08.2013 CN 201310375934
(43) Date of publication of application: 06.07.2016
(73) Proprietor: GiMer Medical Co., Ltd., New Taipei City 251 (TW)
(72) Inventor: CHANG, Chi-Heng, New Taipei City 251 (TW); LIN, Wei-Tso, New Taipei City 251 (TW); CHENG, Chan-Yi, New Taipei City 251 (TW); LIN, Chii-Wann, New Taipei City 251 (TW); WEN, Yeong-Ray, Tamsui Dist., New Taipei City (TW)
(74) Representative: Zimmermann, Tankred Klaus
(86) International application number: PCT/CN2014/081207
(87) International publication number: WO 2015/027748

(56) References cited:
- CN-A- 1 234 744
- CN-A- 1 625 421
- US-A- 4 398 537
- US-A1- 2011 130 804
- US-A1- 2012 035 687
- US-A1- 2012 296 389
- US-A1- 2013 150 919
- US-A1- 2013 317 564
- US-A1- 2015 100 112
- HUNG-WEI CHIU ET AL: "Pain Control on Demand Based on Pulsed Radio-Frequency Stimulation of the Dorsal Root Ganglion Using a Batteryless Implantable CMOS SoC", IEEE TRANSACTIONS ON BIOMEDICAL CIRCUITS AND SYSTEMS, IEEE, US, vol. 4, no. 6, 1 December 2010 (2010-12-01), pages 350-359, XP011336788, ISSN: 1932-4545, DOI: 10.1109/TBCAS.2010.2081668

## Description

### BACKGROUND

### Technical Field

The present invention is generally related to nerve stimulation by electromagnetic field, and more particular to a device of applying electromagnetic stimulation to increase nerve threshold.

### Related Art

The central nervous system provides transmission paths for the commands issued from the brain. The central nervous has a threshold and the threshold is often reduced around a damaged spot of the nerve. Therefore, uncomfortable pain or ache is frequently and easily felt at this spot. After a period of time, this spot would become a source of chronic pain.

Clinically, an approach called Continuous Radiofrequency (CRF) or Radiofrequency Ablation is widely applied to ease various nerve pains. The approach inserts a pin into the proximity of related nerve tissue, applies continuous, high-frequency signal to create high temperature so as to destroy the nerve tissue, thereby alleviating the nerve pain. However, due to the human body's self-repair function, the destroyed nerve tissue will try to heal itself. When this happens, newly developed tissue grows randomly on the destroyed tissue, and it is quite common that a neuroma is formed. The neuroma, once formed, often oppresses the nerve system and causes even more serious pain.

Another conventional clinical approach is the so-called Pulsed Radiofrequency (PRF) where one electrode is inserted into the proximity of a nerve tissue. Then, a radiofrequency signal of 45V is employed so that the nerve tissue is stimulated twice every second, and for 20 ms every stimulation. By using intermittent pulses, the temperature of the stimulation process does not exceed 42°C, avoiding the high temperature's damaging or destroying the nerve tissue, and as such preventing the occurrence of post-operation neuroma.

One example is US 6,246,912 B1 titled "Modulated high frequency tissue modification," which teaches the connection of a high-frequency pulse generator 11 to a positive electrode pole 1 and a negative ground pad 16, the insertion of the electrode pole 1 into human body so that a terminal 2 of the electrode pole 1 is at a nerve spot to be treated, and the attachment of the ground pad 16 externally to the human body forms an electrical loop. Then, the nerve spot is simulated by a train of electrical pulses of a few dozen volts through the terminal 2. In the process, the temperature is controlled so that the nerve tissue is not damaged or destroyed by high temperature. However, this can be achieved only when the duration of the pulses and the intermittent delay between pulses are precisely controlled.

US 2011/130804 A1 describes a method for treating a nervous symptom or condition in a subject with a pulsed-radiofrequency stimulation system with a low voltage to overcome the disadvantages of the known related stimulation systems.

US 2012/035687 A1 describes an implantable electrical stimulator which includes two stimulating electrodes, a system-on-chip and an inductive coil. The system-on-chip can apply electric stimulation to the dorsal root ganglion via the stimulating electrodes. An external power supply can wirelessly charge the system-on-chip through the inductive coil.

HUNG-WEI CHIU ET AL, "Pain Control on Demand Based on Pulsed Radio-Frequency Stimulation of the Dorsal Root Ganglion Using a Batteryless Implantable CMOS SoC", IEEE TRANSACTIONS ON BIOMEDICAL CIRCUITS AND SYSTEMS, IEEE, US, (20101201), vol. 4, no. 6, doi:10.1109/TBCAS.2010.2081668, ISSN 1932-4545, pages 350 - 359, XP011336788, describe the implementation of a batteryless CMOS SoC with low voltage pulsed radio-frequency (PRF) stimulation.

### SUMMARY

It is an object of the present invention to provide an embedded electromagnetic stimulation device to obviate the shortcomings of the prior arts.

The invention is directed to an embedded electromagnetic stimulation device as defined in the appended claims.

By the stimulation of the low-power, low-temperature, high-frequency electromagnetic field, the nerve's threshold is increased, the nerve's transmission capability is reduced, and therefore the nerve pain is effectively eased.

### BRIEF DESCRIPTION OF THE DRAWINGS

FIG. 1 is ac flow diagram showing an operation scenario of an electromagnetic stimulation device according to the present invention.
FIG. 2 is a schematic diagram showing an electromagnetic stimulation device according to the present invention.
FIG. 3 is a graph showing the strength and pattern of the electromagnetic field produced by the electromagnet stimulation device of FIG. 2 where the distance between the device's electrodes is of a shorter distance.
FIG. 4 is a graph showing the strength and pattern of the electromagnetic field produced by the electromagnetic stimulation device of FIG. 2 where the distance between the device's electrodes is of a greater distance.
FIG. 5 is a graph showing the strength of the electromagnetic field produced by the electromagnetic stimulation device of FIG. 2 under various distances between the device's electrodes and the nerve to be stimulated.
FIG. 6 is a schematic diagram showing a first embodiment of the electromagnetic stimulation device of FIG. 2.
FIG. 7 is a schematic diagram showing a second embodiment of the electromagnetic stimulation device of FIG. 2.
FIG. 8 is a schematic diagram showing a third embodiment of the electromagnetic stimulation device of FIG. 2.
FIG. 9 is a schematic diagram showing a fourth embodiment of the electromagnetic stimulation device of FIG. 2.
FIG. 10 is a schematic diagram showing an arrangement of the electrodes of the electromagnetic stimulation device of FIG. 7.
FIG. 11 is a schematic diagram showing another arrangement of the electrodes of the electromagnetic stimulation device of FIG. 7.
FIG. 12 is a graph showing the strength and pattern of the electromagnetic field produced by the electromagnetic stimulation device of FIG. 7.
FIG. 13 is a schematic diagram showing a fifth embodiment of the electromagnetic stimulation device of FIG. 2.

Wherein:
1: electromagnetic stimulation device
11: insulating member
12: positive electrode
13: negative electrode
2: nerve
a: the electrode width of the positive and negative electrodes
b: first distance
c: second distance

### DETAILED DESCRIPTION OF THE INVENTION

The following descriptions are exemplary embodiments only, and are not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention as set forth in the appended claims.

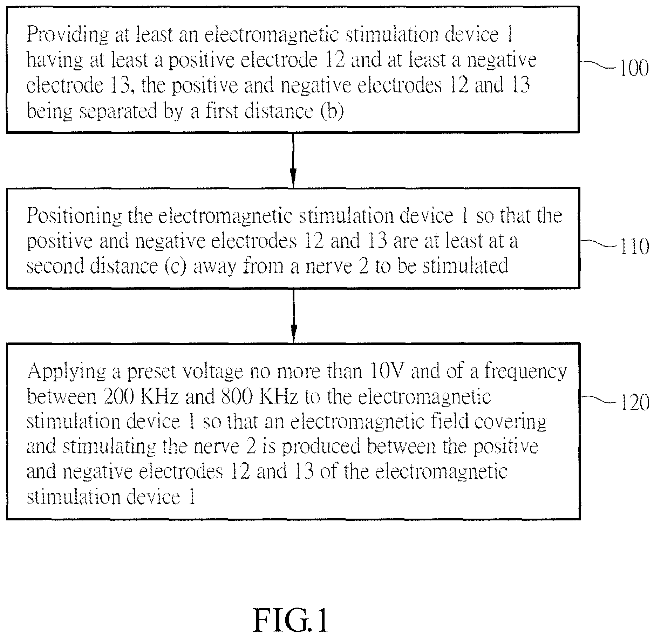

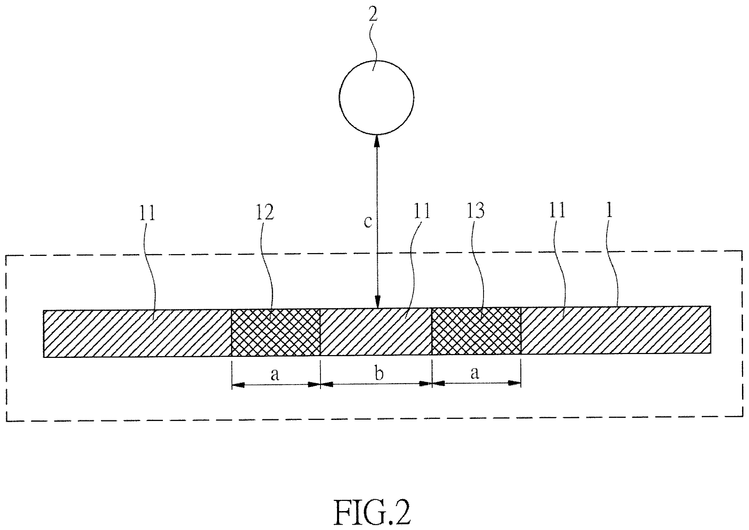

As shown in FIGS. 1 and 2, an electromagnetic stimulation device for changing nerve threshold is described as follows.

The electromagnetic stimulation device 1 contains at least a positive electrode 12 and at least a negative electrode 13 configured on an insulating member 11. The positive and negative electrodes 12 and 13 are separated by an insulating gap having width of a first distance (b). The positive and negative electrodes 12 and 13 are therefore separated by the first distance (b). The first distance (b) is between 3 mm and 7 mm. The positive and negative electrodes 12 and 13 are at least at a second distance (c) away from a nerve 2 to be stimulated. The second distance (c) is no more than 10 mm. A preset voltage is applied to the positive and negative electrodes 12 and 13. The preset voltage is no more than 10V and has a frequency between 200 KHz and 800 KHz. Then, a low-power, low-temperature, high-frequency electromagnetic field covering and stimulating the nerve 2 is produced between the positive and negative electrodes 12 and 13.

Preferably, the preset voltage is between +3V and +10V, or between -3V and -10V, and has a frequency between 200 KHz and 800 KHz.

An embodiment of the present invention is described as follows. The electromagnetic stimulation device 1 is electrically connected to an external power supply which provides the preset voltage of no more than 10V with a frequency between 200 KHz and 800 KHz. A low-power, low-temperature, high-frequency electromagnetic field is produced between the positive and negative electrodes 12 and 13 of the electromagnetic stimulation device 1. Each of the positive and negative electrodes 12 and 13 has a width (a) between 1 mm and 3 mm.

An operation scenario of the electromagnetic stimulation device 1 is described as follows.

In step 100, at least an electromagnetic stimulation device 1 is provided. The electromagnetic stimulation device 1 contains at least a positive electrode 12 and at least a negative electrode 13. The positive and negative electrodes 12 and 13 are separated by an insulating gap having width of a first distance (b). The positive and negative electrodes 12 and 13 are therefore separated by the first distance (b).

In step 110, the electromagnetic stimulation device 1 is positioned so that the positive and negative electrodes 12 and 13 are at least at a second distance (c) away from a nerve 2 to be stimulated.

In step 120, a preset voltage is applied to the positive and negative electrodes 12 and 13. The preset voltage is no more than 10V and has a frequency between 200 KHz and 800 KHz. Then, a low-power, low-temperature, high-frequency electromagnetic field covering and stimulating the nerve 2 is produced between the positive and negative electrodes 12 and 13.

The low-power, low-temperature, high-frequency electromagnetic field covers the nerve 2 so that the nerve 2 is stimulated by the electromagnetic field without damaging the nerve 2's tissue cells. As such, the production of biomolecules from the nerve 2 is inhibited, the nerve threshold around nerve 2 is modified and increased, and the transmission capability around nerve 2 is reduced, effectively lessening the nerve pain of a user.

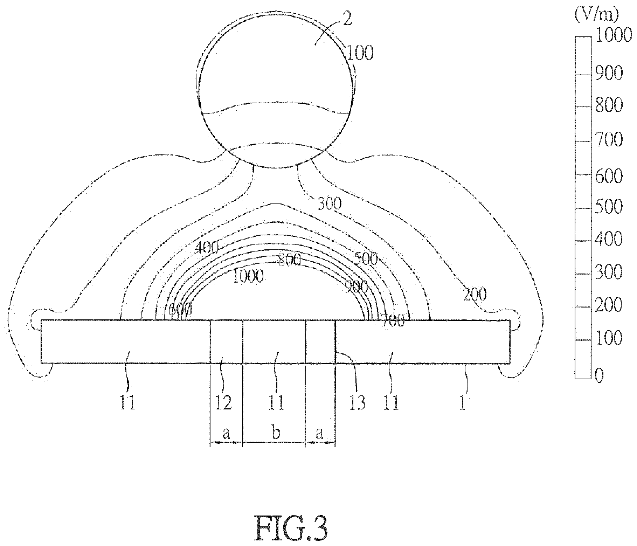

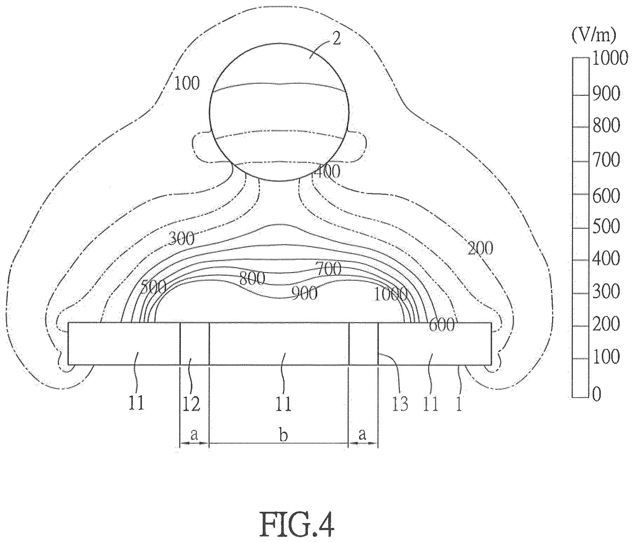

The electromagnetic field's field pattern can be adjusted by selecting appropriate electrode width (a), the first distance (b), and the second distance (c). Another embodiment of the present invention is described as follows. The preset voltage is set to 5V with frequency 500 KHz. The electrode width (a) of the positive and negative electrodes 12 and 13, and the second distance (c) are fixed. Then, as shown in FIG. 3, when the first distance (b) between the positive and negative electrodes 12 and 13 is smaller, the electromagnetic field covers a portion of the to-be-stimulated nerve 2. On the other hand, when the first distance (b) is larger, as shown in FIG. 4, the electromagnetic field entirely covers the nerve 2. Additionally, the strength of the electromagnetic field is stronger as it is closer to the positive and negative electrodes 12 and 13.

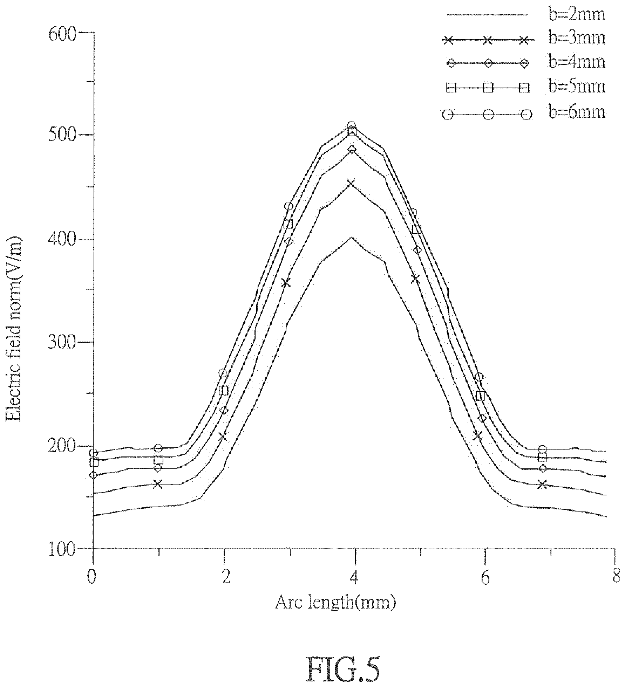

Yet another embodiment of the present invention is described as follows. The preset voltage is set to 5V with frequency 500 KHz. The electrode width (a) of the positive and negative electrodes 12 and 13 is 1 mm, and the second distance (c) is 5 mm. Then, as shown in FIG. 5, where the electromagnetic field is measured when the first distance (b) between the positive and negative electrodes 12 and 13 is set to 2, 3, 4, 5, and 6 mm, respectively, it should be obvious that the electromagnetic field has a stronger strength when the first distance (b) is between 4 mm and 5 mm.

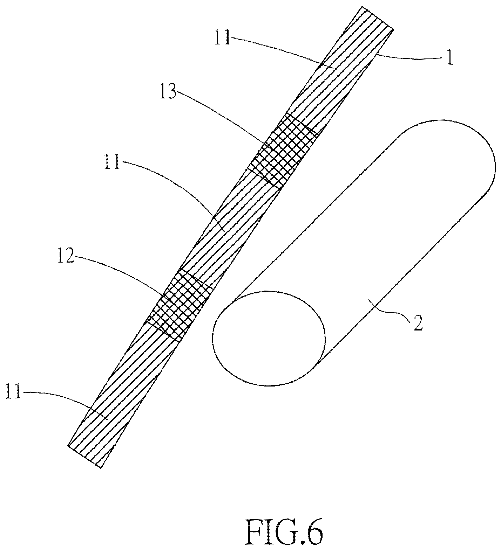

As shown in FIGS. 2 and 6, the electromagnetic stimulation device 1 can have a linear shape. The electromagnetic stimulation device 1 contains at least one positive electrode 12 and at least one negative electrode 13. The positive and negative electrodes 12 and 13 are separated by the first distance (b). Then, the electromagnetic field produced by the positive and negative electrodes 12 and 13 are applied to the nerve 2 to conduct a low-power, low-temperature, high-frequency electromagnetic stimulation.

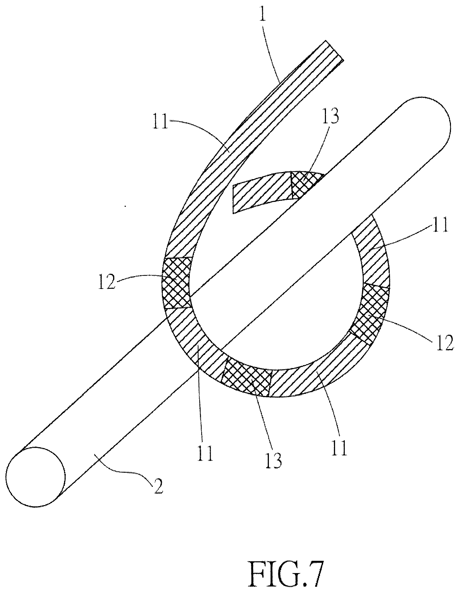

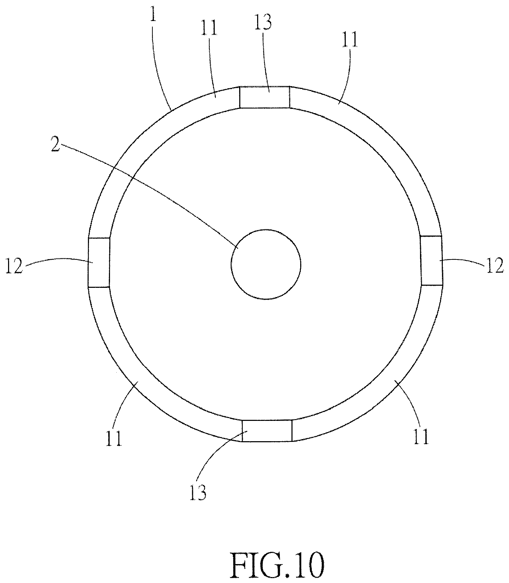

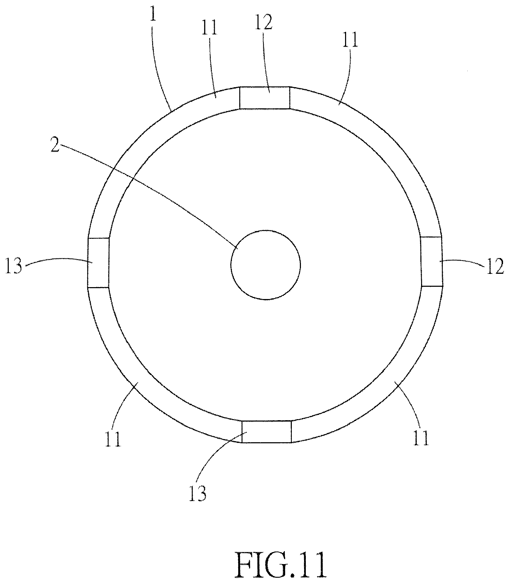

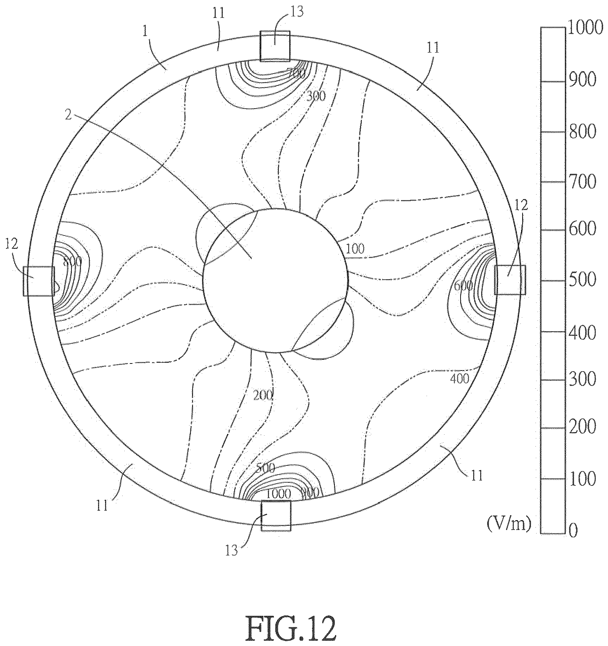

As shown in FIGS. 7, 10, 11, and 12, the electromagnetic stimulation device 1 can have a ring shape. The electromagnetic stimulation device 1 contains at least two positive electrodes 12 and at least two negative electrodes 13, where the positive and negative electrodes 12 and 13 are arranged (e.g., as shown in FIG. 10) alternately around and at least at the second distance (c) from the nerve 2. Alternatively, the positive and negative electrodes 12 and 13 are arranged (e.g., as shown in FIG. 11) sequentially (not alternately) around and at least at the second distance (c) from the nerve 2. Then, as shown in FIG. 12, the electromagnetic field produced by the positive and negative electrodes 12 and 13 are applied to the nerve 2 to conduct a low-power, low-temperature, high-frequency electromagnetic stimulation. Again, the strength of the electromagnetic field is stronger as it is closer to the positive and negative electrodes 12 and 13.

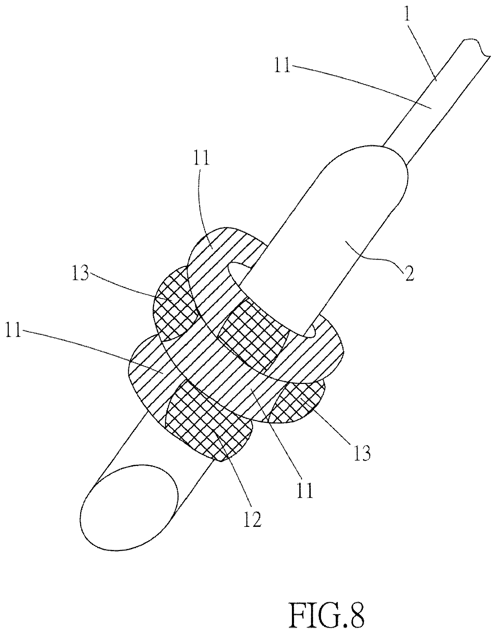

As shown in FIG. 8, the electromagnetic stimulation device 1 can have a helix shape. The electromagnetic stimulation device 1 contains at least two positive electrodes 12 and at least two negative electrodes 13, where the positive and negative electrodes 12 and 13 can be arranged alternately or sequentially around and at least at the second distance (c) from the nerve 2. Then, the electromagnetic field produced by the positive and negative electrodes 12 and 13 are applied to the nerve 2 to conduct a low-power, low-temperature, high-frequency electromagnetic stimulation.

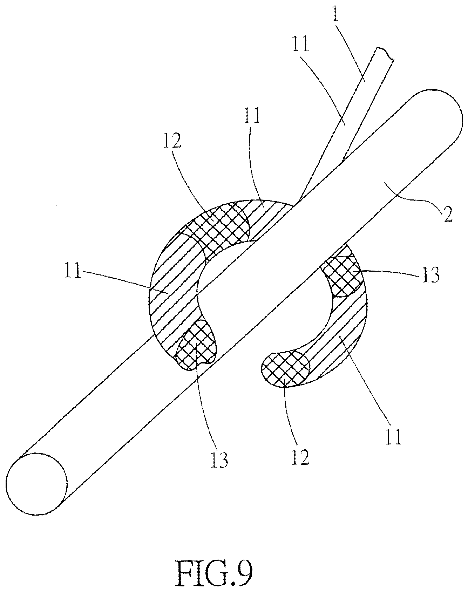

As shown in FIG. 9, the electromagnetic stimulation device 1 can have a C-like shape. The electromagnetic stimulation device 1 contains at least two positive electrodes 12 and at least two negative electrodes 13, where the positive and negative electrodes 12 and 13 can be arranged alternately or sequentially around and at least at the second distance (c) from the nerve 2. Then, the electromagnetic field produced by the positive and negative electrodes 12 and 13 are applied to the nerve 2 to conduct a low-power, low-temperature, high-frequency electromagnetic stimulation.

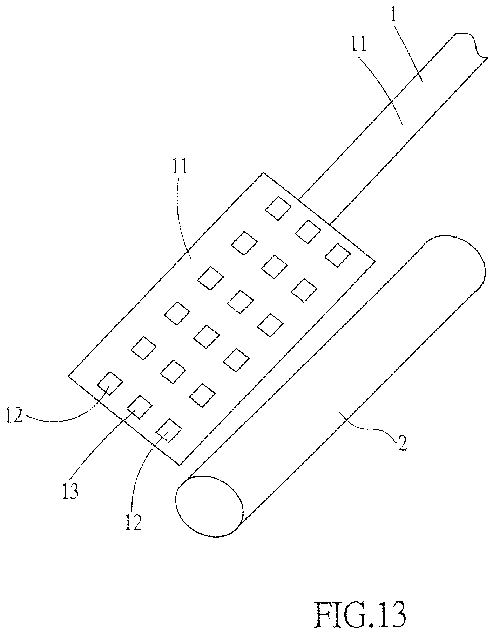

As shown in FIG. 13, the electromagnetic stimulation device 1 can have a planar shape. The electromagnetic stimulation device 1 contains more than two positive electrodes 12 and more than two negative electrodes 13, where the positive and negative electrodes 12 and 13 can be arranged alternately or sequentially in an array at least at the second distance (c) from the nerve 2. Then, the electromagnetic field produced by the positive and negative electrodes 12 and 13 are applied to the nerve 2 to conduct a low-power, low-temperature, high-frequency electromagnetic stimulation.

Please note that electromagnetic stimulation device 1 can be embedded inside or installed outside human body. Either way the positive and negative electrodes 12 and 13 can be arranged alternately or sequentially at least at the second distance (c) from the nerve 2.

## Claims

1. An embedded electromagnetic stimulation device, comprising at least a positive electrode (12) and at least a negative electrode (13), wherein
the positive and negative electrodes (12, 13) are separated by a first distance (b);
the embedded electromagnetic stimulation device is configured to be positioned so that the positive and negative electrodes (12, 13) are at least at a second distance (c) away from a nerve (2) to be stimulated, wherein the second distance (c) is no more than 10 mm;
the embedded electromagnetic stimulation device is configured to have applied a preset voltage to the positive and negative electrodes (12, 13) so that an electromagnetic field covering and stimulating the nerve (2) is produced between the positive and negative electrodes (12, 13); and
the preset voltage is no more than 10V and has a frequency between 200 KHz and 800 KHz,
wherein the electromagnetic stimulation device further comprises an insulating member (11); the positive and negative electrodes (12, 13) are configured on the insulating member (11); and the first distance (b) is between 3 mm and 7 mm.

2. The embedded electromagnetic stimulation device according to claim 1, wherein the preset voltage is between +3V and +10V, or between -3V and -10V; and has a frequency between 200 KHz and 800 KHz.

3. The embedded electromagnetic stimulation device according to claim 1, wherein each of the positive and negative electrodes has a width between 1 mm and 3 mm.

4. The embedded electromagnetic stimulation device according to claim 1, wherein the electromagnetic stimulation device has a linear shape, and comprises at least one positive electrode (11) and at least one negative electrode (12).

5. The embedded electromagnetic stimulation device according to claim 1, wherein the electromagnetic stimulation device has a ring shape, and comprises at least two positive electrodes (12) and at least two negative electrodes (13) to be arranged around and at least at the second distance (c) from the nerve (2).

6. The embedded electromagnetic stimulation device according to claim 1, wherein the electromagnetic stimulation device has a curve shape, and comprises at least two positive electrodes (12) and at least two negative electrodes (13) to be arranged around and at least at the second distance (c) from the nerve (2).

7. The embedded electromagnetic stimulation device according to claim 1, wherein the electromagnetic stimulation device has a helix shape, and comprises at least two positive electrodes (12) and at least two negative electrodes (13) to be arranged around and at least at the second distance (c) from the nerve (2).

8. The embedded electromagnetic stimulation device according to claim 1, wherein the electromagnetic stimulation device comprises a plurality of positive electrodes (12) and a plurality of negative electrodes (13) to be arranged in an array and at least at the second distance (c) from the nerve (2).

9. The embedded electromagnetic stimulation device according to claim 1, wherein the electromagnetic stimulation device has a linear shape, a ring shape, a C-like shape, a helix shape or a planar shape.

## Patentansprüche

1. Eine eingebettete elektromagnetische Stimulationsvorrichtung, die zumindest eine positive Elektrode (12) und zumindest eine negative Elektrode (13) aufweist, wobei
die positive und die negative Elektrode (12, 13) um einen ersten Abstand (b) getrennt sind;
die eingebettete elektromagnetische Stimulationsvorrichtung konfiguriert ist, um derart positioniert zu sein, dass die positive und negative Elektrode (12, 13) zumindest mit einem zweiten Abstand (c) von einem zu stimulierenden Nerv (2) beabstandet sind, wobei der zweite Abstand (c) nicht mehr als 10 mm beträgt;
die eingebettete elektromagnetische Stimulationsvorrichtung derart konfiguriert ist, dass eine voreingestellte Spannung an die positive und negative Elektrode (12, 13) angelegt ist, so dass zwischen der positiven und negativen Elektrode (12, 13) ein elektromagnetisches Feld, das den Nerv (2) bedeckt und stimuliert, erzeugt wird; und
die voreingestellte Spannung nicht mehr als 10V beträgt und eine Frequenz zwischen 200 KHz und 800 KHz aufweist,
wobei die elektromagnetische Stimulationsvorrichtung ferner ein isolierendes Bauglied (11) aufweist; die positive und negative Elektrode (12, 13) auf dem isolierenden Bauglied (11) konfiguriert sind; und der erste Abstand (b) zwischen 3 mm und 7 mm beträgt.

2. Die eingebettete elektromagnetische Stimulationsvorrichtung gemäß Anspruch 1, bei der die voreingestellte Spannung zwischen +3V und +10V oder zwischen -3V und -10V beträgt; und eine Frequenz zwischen 200 KHz und 800 KHz aufweist.

3. Die eingebettete elektromagnetische Stimulationsvorrichtung gemäß Anspruch 1, bei der sowohl die positive als auch die negative Elektrode eine Breite zwischen 1 mm und 3 mm aufweist.

4. Die eingebettete elektromagnetische Stimulationsvorrichtung gemäß Anspruch 1, wobei die elektromagnetische Stimulationsvorrichtung eine lineare Form aufweist und zumindest eine positive Elektrode (11) und zumindest eine negative Elektrode (12) aufweist.

5. Die eingebettete elektromagnetische Stimulationsvorrichtung gemäß Anspruch 1, wobei die elektromagnetische Stimulationsvorrichtung eine Ringform aufweist und zumindest zwei positive Elektroden (12) und zumindest zwei negative Elektroden (13) aufweist, die um den Nerv (2) herum und zumindest mit dem zweiten Abstand (c) von demselben anzuordnen sind.

6. Die eingebettete elektromagnetische Stimulationsvorrichtung gemäß Anspruch 1, wobei die elektromagnetische Stimulationsvorrichtung eine Kurvenform aufweist und zumindest zwei positive Elektroden (12) und zumindest zwei negative Elektroden (13) aufweist, die um den Nerv (2) herum und zumindest mit dem zweiten Abstand (c) von demselben anzuordnen sind.

7. Die eingebettete elektromagnetische Stimulationsvorrichtung gemäß Anspruch 1, wobei die elektromagnetische Stimulationsvorrichtung eine Spiralform aufweist und zumindest zwei positive Elektroden (12) und zumindest zwei negative Elektroden (13) aufweist, die um den Nerv (2) herum und zumindest mit dem zweiten Abstand (c) von demselben anzuordnen sind.

8. Die eingebettete elektromagnetische Stimulationsvorrichtung gemäß Anspruch 1, wobei die elektromagnetische Stimulationsvorrichtung eine Mehrzahl positiver Elektroden (12) und eine Mehrzahl negativer Elektroden (13) aufweist, die in einem Array und zumindest mit dem zweiten Abstand (c) von dem Nerv (2) anzuordnen sind.

9. Die eingebettete elektromagnetische Stimulationsvorrichtung gemäß Anspruch 1, wobei die elektromagnetische Stimulationsvorrichtung eine lineare Form, eine Ringform, eine C-artige Form, eine Spiralform oder eine planare Form aufweist.

## Revendications

1. Dispositif de stimulation électromagnétique incorporé, comprenant au moins une électrode positive (12) et au moins une électrode négative (13), dans lequel
les électrodes positives et négatives (12, 13) sont séparées d'une première distance (b);
le dispositif de stimulation électromagnétique incorporé est configuré pour être positionné de sorte que les électrodes positives et négatives (12, 13) se situent au moins à une deuxième distance (c) d'un nerf (2) à stimuler, où la deuxième distance (c) n'est de pas plus de 10 mm;
le dispositif de stimulation électromagnétique intégré est configuré pour avoir appliqué une tension préréglée aux électrodes positives et négatives (12, 13), de sorte qu'il se produise un champ électromagnétique couvrant et stimulant le nerf (2) entre les électrodes positives et négatives (12, 13); et
la tension préréglée n'est de pas plus de 10V et a une fréquence comprise entre 200 KHz et 800 KHz,
dans lequel le dispositif de stimulation électromagnétique comprend par ailleurs un élément isolant (11); les électrodes positives et négatives (12, 13) sont configurées sur l'élément isolant (11); et la première distance (b) est comprise entre 3 mm et 7 mm.

2. Dispositif de stimulation électromagnétique incorporé selon la revendication 1, dans lequel la tension préréglée est comprise entre +3V et +10V, ou entre -3V et -10V; et a une fréquence comprise entre 200 KHz et 800 KHz.

3. Dispositif de stimulation électromagnétique incorporé selon la revendication 1, dans lequel chacune des électrodes positives et négatives présente une largeur comprise entre 1 mm et 3 mm.

4. Dispositif de stimulation électromagnétique incorporé selon la revendication 1, dans lequel le dispositif de stimulation électromagnétique présente une forme linéaire et comprend au moins une électrode positive (11) et au moins une électrode négative (12).

5. Dispositif de stimulation électromagnétique incorporé selon la revendication 1, dans lequel le dispositif de stimulation électromagnétique présente une forme d'anneau, et comprend au moins deux électrodes positives (12) et au moins deux électrodes négatives (13) destinées à être disposées autour et au moins à la deuxième distance (c) du nerf (2).

6. Dispositif de stimulation électromagnétique incorporé selon la revendication 1, dans lequel le dispositif de stimulation électromagnétique présente une forme courbe, et comprend au moins deux électrodes positives (12) et au moins deux électrodes négatives (13) destinées à être disposées autour et au moins à la deuxième distance (c) du nerf (2).

7. Dispositif de stimulation électromagnétique incorporé selon la revendication 1, dans lequel le dispositif de stimulation électromagnétique présente une forme hélicoïdale, et comprend au moins deux électrodes positives (12) et au moins deux électrodes négatives (13) destinées à être disposées autour et au moins à la deuxième distance (c) du nerf (2).

8. Dispositif de stimulation électromagnétique incorporé selon la revendication 1, dans lequel le dispositif de stimulation électromagnétique comprend une pluralité d'électrodes positives (12) et une pluralité d'électrodes négatives (13) destinées à être disposées selon un réseau et au moins à la deuxième distance (c) du nerf (2).

9. Dispositif de stimulation électromagnétique incorporé selon la revendication 1, dans lequel le dispositif de stimulation électromagnétique présente une forme linéaire, une forme d'anneau, une forme de "C", une forme hélicoïdale ou une forme plane.
